# EUROPEAN PATENT SPECIFICATION

(11) **EP 1 362 590 B1**
(45) Date of publication and mention of the grant of the patent: **07.01.2004**
(21) Application number: 02016602.1
(22) Date of filing: 25.07.2002
(51) Int. Cl.: A61K 31/55, A61P 9/04

(54) **Use of cilobradine or the pharmaceutically acceptable salts thereof for the treatment or prevention of heart failure**
Verwendung von Cilobradine oder pharmazeutisch akzeptablen Salze zur Behandlung oder Prävention von Herzversagen
Utilisation de cilobradine et leurs sels pharmaceutiquement accceptables pour le traitement ou la prévention de l'insuffisance cardiaque

(43) Date of publication of application: 19.11.2003
(73) Proprietor: Boehringer Ingelheim Pharma GmbH & Co.KG, 55218 Ingelheim am Rhein (DE)
(72) Inventor: Dr. Brian GUTH, 88447 Warthausen (DE); Dr. Randolf SEIDLER, Sandy Hook, CT 06482 (US); Dr. Jürgen DÄMMGEN, 88416 Ochsenhausen (DE)

(56) References cited:
- EP-A- 0 224 794
- EP-A- 0 330 052
- EP-A- 0 471 388
- WO-A-01/78699
- SHINKE T ET AL: "BENEFICIAL EFFECTS OF HEART RATE REDUCTION ON CARDIAC MECHANICS ANDENERGETICS IN PATIENTS WITH LEFT VENTRICULAR DYSFUNCTION" JAPANESE CIRCULATION JOURNAL, KYOTO, JP, vol. 63, no. 12, December 1999 (1999-12), pages 957-964, XP001041863 ISSN: 0047-1828

## Description

### FIELD OF THE INVENTION

The present invention relates to the use of cilobradine, or the pharmaceutically acceptable salts thereof, for the treatment or prevention of heart failure of any aetiology.

### BACKGROUND OF THE INVENTION

Heart failure is a major world-wide public health problem and is the only cardiac disorder that is increasing in incidence. In the United States alone, 5 million patients suffer from heart failure, with a new diagnosis made in 0.5 million patients per year. Despite advances in therapy, over the last decade, the annual number of hospitalisations has increased from 550 000 to 900 000 as a primary diagnosis, and from 1.7 to 2.6 million as a primary or secondary diagnosis (J. Am. Pharm. Assoc., vol. 41 (5), pp. 672-681, 2001). Unless treated, heart failure may lead to death. Hence, new approaches are warranted to treat or prevent heart failure.

Although the terminology heart failure seems to be the most accepted terminology for describing this cardiac disorder, various further equivalent terminologies can be found in the scientific, patent or medical literature as, for example, cardiac failure, insufficient cardiac output, cardiac insufficiency, cardiac collapse and cardiac syncope.

Furthermore, though heart failure is invariably a chronic cardiac disorder, often with an insidious onset, heart failure may be present acutely or be punctuated by episodes of acute deterioration, so called "decompensated" heart failure. To describe these conditions also related to heart failure, further terminologies will commonly be found in the scientific, patent or medical literature such as, for example, chronic heart failure, acute heart failure, heart decompensation, cardiac decompensation and cardial decompensation.

Lastly, as will be explained in the foregoing, as heart failure can be caused by a dysfunctioning of the heart reflected by various clinical presentations and sometimes subjected to further complications, further terminologies related to heart failure will also commonly be found in the scientific, patent or medical literature such as, for example, myocardial failure, myocardial insufficiency, heart muscle insufficiency, cardiac muscle insufficiency, heart muscle weakness, cardiac muscle weakness, systolic or left ventricular heart failure, diastolic heart failure, left or right sided heart failure, biventricular heart failure and congestive heart failure.

Hence, a distinction can be made between the systolic or diastolic origin of the dysfunctioning. Commonly, heart failure is a consequence of a progressive deterioration of myocardial contractile function, named systolic or left ventricular dysfunction. However, diastolic dysfunction is becoming increasingly recognised as an important cause of heart failure too. This occurs when the heart chambers are unable to expand sufficiently during diastole (period of heart relaxation in which the chambers fill with blood) and hence blood volume in the ventricles is inadequate. Whether systolic and/or diastolic dysfunction is the basis of heart failure, cardiac output is diminished. When additionally there is "damming" back of blood in the venous system, congestion may ensue in the lungs (pulmonary oedema) and/or in the abdomen or peripheries (peripheral oedema). When both occur, the terminology congestive heart failure is often used.

In other respects, the distinction between left and right sided heart failure can be applied to reflect the clinical presentation (i.e. pulmonary oedema indicative of left sided heart failure, whereas the principal symptom of right sided heart failure is fluid retention in the peripheries) or to denote the underlying cause. Right sided heart failure is most commonly a consequence of left sided heart failure, although diseases of the lung (such as chronic obstructive pulmonary disease), the right ventricle (e.g. right ventricular infarction) or the vasculature (primary or secondary pulmonary hypertension, the latter due to conditions such as pulmonary embolism for example), may result in predominate right sided heart failure.

According to the International Classification of Functioning, Disability and Health, lastly published by the World Health Organization on 15 November 2001 (ISBN 91 4 1545429) and accepted by 191 countries during the 54^{th} World Health Assembly (Resolution WHA 54.21), heart failure occurs when the heart function of pumping the blood in adequate or required amounts and pressure throughout the body is impaired.

As cardiac output is normally 5 litres/minute, although this can increase five fold with heavy exercise, in essence, heart failure occurs when the heart is unable to meet this demand.

As heart failure manifests itself in a variety of ways, at the time of this patent application, the treatment or prevention of heart failure comprises a combination of typical medications. These medications are based upon the principles of promoting fluid excretion to lessen oedema and volume overload (e.g. a various types of diuretics), vasodilatory drugs to reduce preload (i.e. atrial pressures) and/or afterload (i.e. pressure against which the heart has to beat), and inotropic drugs to increase contractility.

Vasodilatory drugs available at this time include Angiotensin Converting Enzyme (ACE) inhibitors, Angiotensin II Receptor blockers (ARBs) and nitrate venodilators. Inotropic drugs are usually administered only in acute situations. Although cardiac glycosides such as digoxin are sometimes prescribed for their inotropic properties, their use is more common in heart failure patients when atrial arrhythmias co-exist.

Recently, beta-blockers, which were once thought to be contra-indicated in heart failure per se due to their negative inotropic (decreased contractility) property, have been shown to be effective in the treatment of heart failure. Meta-analyses of randomised controlled trials have shown that, in addition to established background therapy of ACE inhibitors and diuretics with or without digoxin, a reduction of all cause mortality and cardiovascular morbidity is conferred by beta-blockers such as carvedilol, metoprolol or bisoprolol (Brophy J. M. et al., Ann. Intern. Med. 2001, Vol. 134, pp. 550-560; Lechat P. et al., Circ. 1998, pp. 1184-1191; Heidenreich P. A. et al., J. Am. Coll. Cardiol., 1997, Vol. 30, pp 27-34).

As heart failure progresses, heart failure treatment is also usually not limited to one single therapy. Hence, add-on therapy use is disclosed for carvedilol, for example, in WO 96/24348, for decreasing the mortality of patients suffering from congestive heart failure. WO 96/40258 discloses a combination therapy comprising an angiotensin II antagonist and spironolactone, an aldosterone receptor antagonist, for the treatment of hypertension, congestive heart disease, cirrhosis and ascites. WO 00/02543 discloses a combination therapy comprising an angiotensin II antagonist (valsartan) and a calcium channel blocker (amlodipine or verapamil) for the treatment of several heart diseases, amongst which acute and chronic congestive heart diseases are cited.

However, as with all therapies, there are constraints to their use. For example, beta-blockers may be contra-indicated in patients with concomitant diseases such as asthma, peripheral vascular disease and decompensated heart failure. Certain drug classes may not be tolerated due to unwanted side effects, e.g. cough with ACE inhibitors, fatigue, dizziness or impotence in association with beta-blockers, and hyponatraemia with diuretics. Furthermore, a slow and careful titration period may be required upon drug initiation, as with beta-blockers, where if not performed, the initial negative effects on the heart's pumping action (negative inotropy) may result in drug intolerance and deterioration in heart failure status.

Hence, to echo the statement set out at the beginning of this section, despite the advances made by therapies established at this time, there is still a need to reduce the unacceptable burden of heart failure and new additional approaches to treatment and prevention of disease progression should be sought.

In searching for new therapies for heart failure, the underlying pathophysiology of the failing heart needs to be considered. It has long been observed in the failing heart that heart rate and contractility are increased short term in order to maintain cardiac performance. In the long term, this response is ultimately damaging. It is for example acknowledged that increased heart rate is a risk factor for mortality and morbidity with adverse consequences on vascular function, atherogenesis, myocardial ischaemia, myocardial energetics and left ventricular function. Chronic tachyarrhythmias are a cause of reversible cardiomyopathy in humans and rapid atrial pacing is established as an animal model of cardiomyopathy. In chronic heart failure, excess adrenergic stimulation signals adverse biological responses (including increased heart rate) via β1, β2 and α2 receptors in the myocardium.

In the failing heart, maintenance of adequate ventricular contraction is sought, but occurs at the expense of oxygen and energy consumption by the myocardium. Heart rate influences such energy demand, with increased heart rate requiring greater expenditure of energy and vice-versa. Thus, greater energetic efficiency could potentially result if heart rate were lowered in heart failure patients.

It thus follows that drugs which have the ability to reduce heart rate may be of benefit in the treatment or prevention of heart failure. For the treatment of cardiac insufficiency, a term also used to denote heart failure, EP 0 471 388 (and its US counterpart US Patent 5,516,773) suggests the use of a specific group of compounds derived from the benzazepine basic chemical structure, and more specifically the compound named zatebradine [1-(7,8-dimethoxy-1,3,4,5-tetrahydro-2H-3-benzazepin-2-one-3-yl)-3-[N-methyl-N-(2-(3,4-dimethoxy-phenyl)-ethyl)-propane].

These benzazepine derivatives were firstly described in EP 0 065 229, as well as their ability to reduce heart rate (bradycardic effect) by acting directly on the sinoatrial node, and their ability to reduce the oxygen requirement of the heart. Zatebradine is also known from WO 01/78699 for the treatment and induction of the regression of idiopathic hypertrophic cardiomyopathy (HCM), ischemic cardiomyopathy and valvular hypertrophic heart diseases.

The effects of the bradycardic agent zatebradine have been studied in a small number of patients with heart failure, also subject to no therapy or atrial pacing, to induce a tachycardia (Shinke et al., Jpn. Circ. Journal, 1999, Vol. 63, pp. 957-964) or in comparison to the beta-blocker propranolol (Shinke et al. Abstract Circ., 1997, Vol. 96, I-644).

In the former study, it was concluded by the authors that the oxygen saving effect of the bradycardia due to zatebradine treatment could be beneficial for the treatment of heart failure. In the latter study, the comparable heart rate reduction observed with zatebradine and the beta-blocker had favourable effects compared to pre-treatment. However, it should be noted that under beta-blocker treatment overall cardiac efficiency was preserved, since the energy saving benefits of heart rate reduction remedied the observed negative effect on contractility. This, the authors proposed, might account for good beta-blockers tolerance and possible efficacy in heart failure. Zatebradine treatment however improved cardiac efficiency since heart rate reduction occurred, but with no accompanying adverse effect on contractility.

It should be noted that these two studies are small and do not attempt to evaluate the benefits of chronic zatebradine administration on the haemodynamic or clinical manifestations of heart failure. Furthermore, the relationships between heart rate reduction, left ventricular function and prognosis in heart failure are complex. However, there is a scientific rationale that improved cardiac energetics secondary to heart rate reduction is an important concept in the treatment and prevention of the progression of heart failure due to systolic and/or diastolic dysfunction (Laperche et al., Heart 1999, Vol. 81, pp. 336-341).

Another specific group of compounds derived from a basic cyclic amine chemical structure, have been shown to also have valuable pharmacological bradycardic properties. These compounds, the process for their preparation and pharmaceutical compositions containing them are described in EP 0 224 794 and its US counterpart US Patent 5,175,157.

One of these cyclic amine derivatives, 3-[(N-(2-(3,4-dimethoxy-phenyl)-ethyl)-piperidin-3-yl)-methyl]-7,8-dimethoxy-1,3,4,5-tetrahydro-2H-3-benzazepin-2-one, and more particularly its S-(+) enantiomer named cilobradine [(+)-3-[(N-(2-(3,4-dimethoxy-phenyl)-ethyl)-piperidin-3-(S)-yl)-methyl]-7,8-dimethoxy-1,3,4,5-tetrahydro-2H-3-benzazepin-2-one], is also known from WO 01/78699 for the treatment and induction of the regression of idiopathic hypertrophic cardiomyopathy (HCM), ischemic cardiomyopathy and valvular hypertrophic heart diseases.

However, cilobradine, has not been suggested for the treatment or prevention of heart failure.

Scientific studies performed with zatebradine and cilobradine in order to determine the mechanism of action of these bradycardic substances have shown that both zatebradine and cilobradine selectively block the hyperpolarisation activated cation current channel (HCN) in cardiac conductive tissue, also referred to as the I_{f} channel. It is through blockade of this channel that zatebradine and cilobradine are assumed to produce their specific bradycardic effect.

However, HCN channels are widely distributed in the nervous system, and in the eye referred to as Iₕ channel. The effect of zatebradine and cilobradine on the Iₕ channel has also been investigated (Neuroscience, Vol. 59(2), pp. 363-373, 1994 for zatebradine, and British Journal of Pharmacology, Vol. 125, pp. 741-750, 1998 for cilobradine). The results have suggested that although Iₕ can also be blocked by these compounds, the interaction with the channels is somewhat different for both tissues. Since Iₕ has been described in the different neurones of the visual signal processing system, the effect on the Iₕ channel has been suggested to be an explanation for the side-effects (visual disturbances) seen by patients treated with I_{f} channel blockers.

Further studies have been performed using electroretinogram (ERG) responses recorded from cat eyes and psychophysical measurements conducted on volunteer human subjects, in normal conditions and after administration of zatebradine (Archives Italiennes de Biologie, vol. 137, pp. 299-309, 1999, and Vision Research, vol. 39, pp. 1767-1774, 1999). The results of these studies have shown that zatebradine reduces the amplitude of the response to stimuli of frequency above 1 Hz and shift the corresponding phase lags, as shown by the ERG recordings. Furthermore, the measurement of the attenuation and phase characteristics of the first harmonic constructed by plotting the response amplitude and the phase as a function of the temporal frequency of the stimulus in control conditions and after intravenous injection or oral administration of zatebradine have shown that the main effect of the Iₕ blocker zatebradine is to decrease the response amplitude to stimuli in the frequency range of 2 to 15 Herz, by introducing a cut-off in the band-pass at about 2 Herz.

To confirm these assumptions, recent studies have been performed using intraretinal and vitreal electroretinogram (ERG) recordings in dark-adapted intact cat retina (Visual Neuroscience, vol. 18(3), pp. 353-363, 2001). These studies compared the changes in the recovery phase following the a- and b-waves induced by an exposure with bright flashes of diffuse white light, after intraretinal injections of substances known to block the responses of bipolar and horizontal cells, or substances known to block Iₕ. The authors of this study have concluded that blockers of Iₕ reduce the recovery phase following the a-wave induced by the light exposure.

### SUMMARY OF THE INVENTION

From the results of the recently published scientific studies on the mechanism of action of bradycardic substances, which were discussed in the previous section, one would not expect an advantage of cilobradine over zatebradine in the treatment of cardiac disorders such as heart failure.

However, as shall be discussed below, it has surprisingly been found that cilobradine presents an advantage over zatebradine not only in terms of its pharmacologically longer duration of action and dose for dose potency, but more importantly in its cardioselectivity, resulting in decreased or absent visual side effects when compared to therapeutic doses of zatebradine.

Hence, a first object of the present invention is that cilobradine has intrinsically different pharmacological properties than zatebradine, which permit full cardiac ion channel blockade with absent or diminished retinal effects. This unexpected cardioselective property represents a clear advantage for cilobradine over, for example, zatebradine, for the treatment of cardiac disorders such as heart failure.

A further object of the present invention is that cilobradine is effective for the treatment or prevention of heart failure of any aetiology and thus, is able to reduce the mortality and morbidity associated with heart failure of any aetiology.

Thus, the present invention is directed to the use of cilobradine, or its pharmaceutically acceptable salts, for the treatment or prevention of heart failure of any aetiology.

### DESCRIPTION OF PREFERRED EMBODIMENTS

In accordance with one embodiment, the present invention provides for a novel use of the cyclic amine derivative (+)-3-[(N-(2-(3,4-dimethoxy-phenyl)-ethyl)-piperidin-3-(S)-yl)-methyl]-7,8-dimethoxy-1,3,4,5-tetrahydro-2H-3-benzazepin-2-one, named cilobradine, or its pharmaceutically acceptable salts.

For the preparation of cilobradine or the pharmaceutically acceptable salts of cilobradine, reference is made to EP 0 224 794 and its US counterpart US Patent 5,175,157, which describes the chemical synthesis of these compounds.

In accordance with a further embodiment of the present invention, amongst the pharmaceutically acceptable salts of cilobradine described in EP 0 224 794 and its US counterpart US Patent 5,175,157, the hydrochloride and hydrobromide salts of cilobradine are preferred.

More particularly, the present invention is directed to the use of cilobradine, or its pharmaceutically acceptable salts, for the preparation of a pharmaceutical composition for the treatment or prevention of heart failure of any aetiology.

In accordance with a further embodiment, the present invention is directed to the use of cilobradine, or its pharmaceutically acceptable salts, for the preparation of a pharmaceutical composition for the prevention of heart failure of any aetiology.

In accordance with a further embodiment of the present invention, the treatment or prevention of heart failure may be assessed by the ability of the compound or pharmaceutical composition in accordance with the present invention to reduce the mortality and morbidity associated with heart failure of any aetiology.

In accordance with a further embodiment of the present invention, the treatment or prevention of heart failure also comprises the treatment or prevention of cardiac insufficiency, cardiac failure, heart insufficiency, myocardial failure, myocardial insufficiency, heart muscle insufficiency, cardiac muscle insufficiency, insufficient cardiac output, heart muscle weakness, cardiac muscle weakness, cardiac collapse, cardiac syncope, chronic heart failure, acute heart failure, heart decompensation, cardiac decompensation, cardial decompensation, diastolic heart failure, right sided heart failure, systolic heart failure, left ventricular heart failure, left sided heart failure, biventricular heart failure and congestive heart failure.

In accordance with a further embodiment of the present invention, heart failure of any aetiology means heart failure diagnosed as a consequence or complication of any other condition, disease or disorder such as, for example, systolic dysfunction, diastolic dysfunction, ischaemic heart diseases, including myocardial infarction, right ventricular infarction and chronic ischaemia, coronary heart diseases, hypertension, primary pulmonary hypertension, secondary pulmonary hypertension, pulmonary embolism, pulmonary arterial stenosis, chronic obstructive pulmonary disease, restrictive cardiomyopathies, dilated cardiomyopathies due to infectious, toxic, metabolic, familial or unknown reasons, myocarditis, congenital anomalies, tachycardias and ventricular hypertrophy secondary to genetic or valvular disorders such as tricuspid valve insufficiency, mitral and/or aortic valve disorders, heart infarcts, thyroid diseases and anaemia.

In accordance with a further embodiment, for the treatment or prevention of heart failure, a combination of cilobradine, or its pharmaceutically acceptable salts, with other substances such as, for example, diuretics, cardiac glycosides, ACE (Angiotensin Converting Enzyme) inhibitors, ARBs (Angiotensin Receptor Blockers), vasodilators, beta blockers and inotropes, present in the same pharmaceutical composition, or given as separate therapies (so-called adjunctive therapy), is also within the scope of the present invention.

In accordance with a further embodiment of the present invention, the pharmaceutical composition for use in accordance with the present invention, comprising cilobradine or its pharmaceutically acceptable salts, alone or in combination with other heart failure therapies including ACE inhibitors, ARBs, diuretics or cardiac glycosides, may be administered to patients in any medically acceptable manner.

In accordance with a further embodiment of the present invention, the pharmaceutical composition for use in accordance with the present invention, comprising cilobradine or its pharmaceutically acceptable salts, may be formulated as liquid formulation or lyophilised powder for oral or parenteral administration. Powders may be reconstituted by addition of a suitable diluent or other pharmaceutically acceptable carrier prior to use. The liquid formulation is generally an aqueous solution. Such formulation is especially suitable for oral administration, but may also be used for parenteral administration or contained in a metered dose inhaler or nebulizer for insufflation. It may be desirable to add excipients such as polyvinylpyrrolidone or hydroxycellulose to the composition.

In accordance with a further embodiment of the present invention, the liquid formulation may be administered directly per orally or filled into a soft capsule.

Alternatively, the ingredients may be encapsulated, tableted or prepared in a syrup for oral administration. Pharmaceutically acceptable solid or liquid carriers may be added to enhance or stabilise the composition, or to facilitate the preparation of the composition. The carrier may also include a sustained release material.

In accordance with a further embodiment of the present invention, the pharmaceutical compositions are prepared following the conventional techniques of pharmacy involving milling, mixing, granulating, and compressing, when necessary, for tablet forms, or milling, mixing and filling for capsule forms.

For the preparation of pharmaceutical compositions comprising cilobradine or its pharmaceutically acceptable salts, reference is made in particular to EP 0 224 794 and its US counterpart US Patent 5,175,157 and to WO 01/78699, which describe examples of injectable, oral liquid, tablet, capsule and suppository formulations of cilobradine or its pharmaceutically acceptable salts.

In accordance with a further embodiment of the present invention, the preferred galenical formulation is a tablet or liquid drinking solution, although capsule, suppository and injectable formulations of the active substance cilobradine or its pharmaceutically acceptable salts are also comprised within the scope of the present invention.

In accordance with a further embodiment of the present invention, the pharmaceutical composition comprising the active compound cilobradine or its pharmaceutically acceptable salts can be administered to animals as well as humans.

In accordance with a further embodiment of the present invention, the pharmaceutical composition comprising the active compound cilobradine or its pharmaceutically acceptable salts is preferably administered following a single or multiple stage daily application scheme.

In accordance with a further embodiment of the present invention, when administered for the treatment or prevention of heart failure, preferably a dose of 0.01 to 20 mg/kg body weight of the active substance cilobradine or its pharmaceutically acceptable salts is used, and this in one or more applications per day. Within this range, the following dose ranges are further preferred: 0.05 to 5 mg/kg body weight, 0.1 to 2.5 mg/kg body weight, 0.1 to 1 mg/kg body weight, and 0.1 to 0.75 mg/kg body weight.

The invention will now be described in more detail with reference to the following example.

As already mentioned above, previous studies (published in Archives Italiennes de Biologie, vol. 137, pp. 299-309, 1999, and Vision Research, vol. 39, pp. 1767-1774, 1999) have established an experimental animal model to evaluate the side-effects (visual disturbances) seen by patients treated with bradycardic agents, such as zatebradine.

These studies were based on a measurement of the electroretinogram (ERG) responses recorded from cat eyes, in normal conditions and after administration of zatebradine.

In the following experiment, the same experiment was performed using cilobradine, and the results compared to the results obtained with zatebradine.

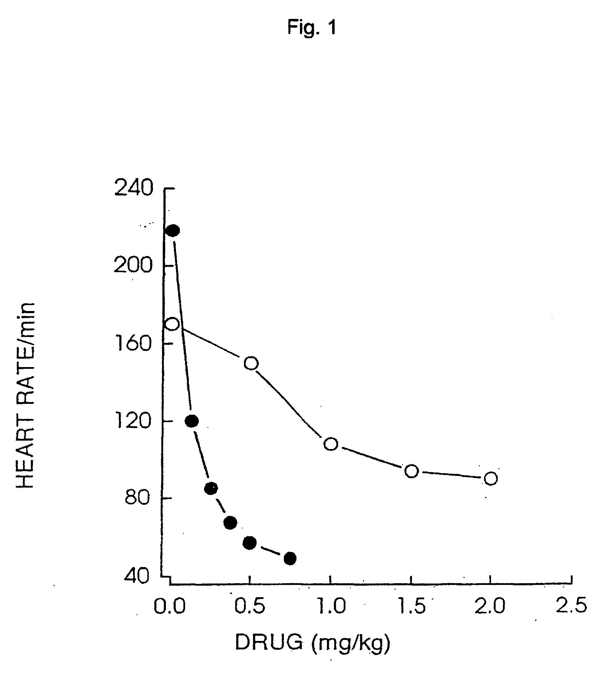

In order to compare the visual side-effect of both compounds in conditions in which the drugs are pharmacologically the most effective in these experiments, as for example in the reduction of heart rate, a dose of 0.75 mg/kg body weight was chosen for cilobradine and a dose of 2.5 mg/kg body weight was chosen for zatebradine. This selection of the dose is based on the result shown in Figure 1, wherein the reduction of heart rate is plotted against the applied dose of the drug (open circles: zatebradine; filled circles: cilobradine). As can be seen from Figure 1, at a dose of 2.5 mg/kg body weight, a reduction of about 44% of the heart rate is obtained with zatebradine (maximum effect), and at a dose of 0.75 mg/kg body weight, a reduction of about 75% of the heart rate is obtained with cilobradine (also maximum effect). Therefore, by choosing these doses, it can already be assumed that the pharmacological effect of cilobradine is better than the pharmacological effect of zatebradine.

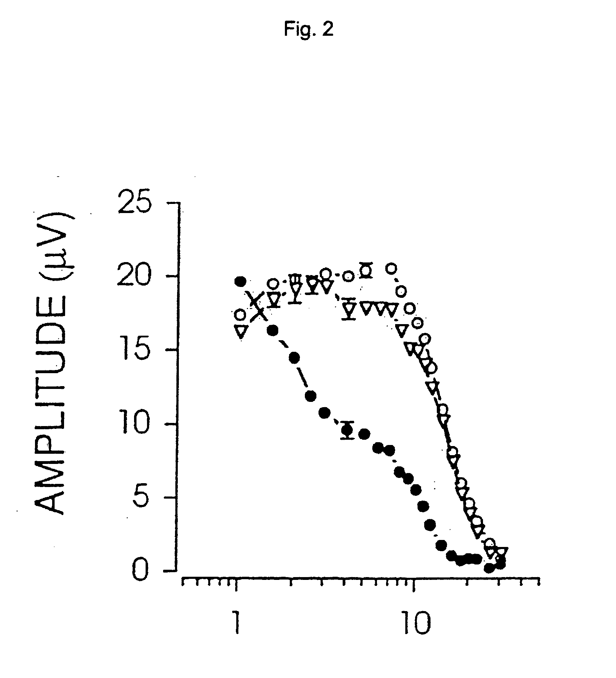

In a similar experiment than the experiment performed by Gargini et al. (published in Vision Research, vol. 39, pp. 1767-1774, 1999), the attenuation and phase characteristics of the ERG response to sinusoidally modulated luminances was evaluated by plotting the amplitude of the response to the light stimulus as a function of the temporal frequency of the light stimulus. The result of this experiment is shown in Figure 2, wherein the open circles are the control responses (no active substance injected), the filled circles are the responses 15 minutes after treatment with a dose of zatebradine of 2.5 mg/kg body weight (i.v. injection), and the triangles are the responses measured 5 hours after the injection of zatebradine.

The results confirm the results already published by Gargini et al. (Vision Research, vol. 39, pp.1767-1774, 1999) that, at this dose, zatebradine reduces the amplitude of the response to stimuli of frequency above 1 Hz and shift the corresponding phase lags, as shown by the ERG recordings. The measurements performed after 5 hours confirm that the visual response is back to normal after 5 hours, and that the experiment is non -destructive for the system.

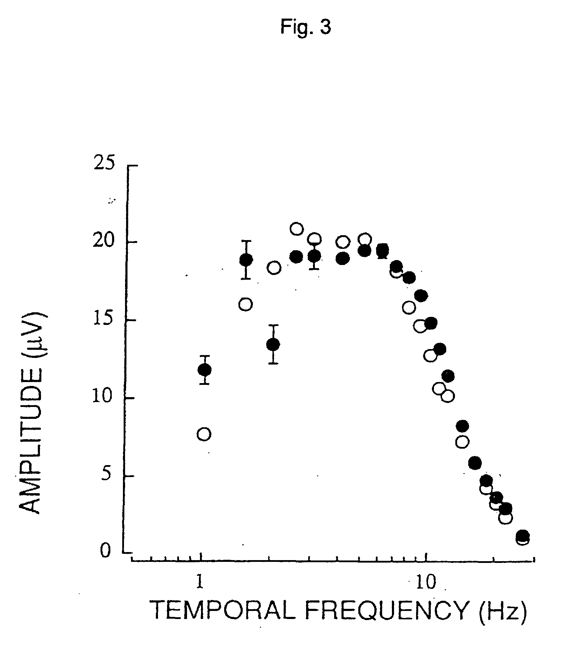

Figure 3 shows the results of the same experiment performed after injection of 0.75 mg/kg body weight of cilobradine, in the same conditions. As is clear from the result, no visual effect can be detected with cilobradine when injected in a fully heart rate reduction effective dose.

We conclude from these results that a dose of cilobradine which produces a saturation effect on the heart rate has negligible consequences on the visual response. This evidences the advantage of cilobradine over zatebradine to produce a pharmacological effect with less side-effect, and thus its superiority for the treatment of heart failure.

## Claims

1. Use of cilobradine or a pharmaceutically acceptable salt thereof for the preparation of a pharmaceutical composition for the treatment or prevention of heart failure.

2. Use in accordance with claim 1, wherein the pharmaceutical composition is for the prevention of heart failure.

3. Use in accordance with claim 1, wherein the pharmaceutical composition is for the treatment or prevention of diastolic heart failure.

4. Use in accordance with claim 3, wherein the pharmaceutical composition is for the prevention of diastolic heart failure.

5. Use in accordance with claim 1, wherein the pharmaceutical composition is for the treatment or prevention of systolic heart failure.

6. Use in accordance with claim 5, wherein the pharmaceutical composition is for the prevention of systolic heart failure.

7. Use in accordance with any one of claims 1 to 6, wherein the galenical formulation of the pharmaceutical composition is a tablet, a drinking solution, a capsule, a suppository or an injectable formulation.

8. Use in accordance with any one of claims 1 to 7, wherein the galenical formulation of the pharmaceutical composition is a tablet.

9. Use in accordance with any one of claims 1 to 7, wherein the galenical formulation of the pharmaceutical composition is a drinking solution.

10. Use in accordance with any one of claims 1 to 9, wherein the pharmaceutical composition is to be administered following a single or multiple stage daily application scheme.

11. Use in accordance with claim 10, wherein cilobradine or its pharmaceutically acceptable salt is to be administered between 0.01 and 20 mg/kg body weight.

12. Use in accordance with claim 10, wherein cilobradine or its pharmaceutically acceptable salt is to be administered between 0.05 and 5 mg/kg body weight.

13. Use in accordance with claim 10, wherein cilobradine or its pharmaceutically acceptable salt is to be administered between 0.1 and 2.5 mg/kg body weight.

14. Use in accordance with claim 10, wherein cilobradine or its pharmaceutically acceptable salt is to be administered between 0.1 and 1 mg/kg body weight.

15. Use in accordance with claim 10, wherein cilobradine or its pharmaceutically acceptable salt is to be administered between 0.1 and 0.75 mg/kg body weight.

16. Use in accordance with any one of claims 1 to 15, wherein the treatment or prevention of heart failure is performed in combination with other therapeutic agents for the treatment or prevention of heart failure, such as diuretics, cardiac glycosides, ACE inhibitors, ARBs, vasodilators, beta blockers or inotropes.

## Patentansprüche

1. Verwendung von Cilobradin oder eines pharmazeutisch annehmbaren Salzes davon zur Herstellung einer Arzneimittelzusammensetzung zur Behandlung oder Vorbeugung von Herzversagen.

2. Verwendung nach Anspruch 1, bei welcher die Arzneimittelzusammensetzung zur Vorbeugung von Herzversagen bestimmt ist.

3. Verwendung nach Anspruch 1, bei welcher die Arzneimittelzusammensetzung zur Behandlung oder Vorbeugung von diastolischem Herzversagen bestimmt ist.

4. Verwendung nach Anspruch 3, bei welcher die Arzneimittelzusammensetzung zur Vorbeugung von diastolischem Herzversagen bestimmt ist.

5. Verwendung nach Anspruch 1, bei welcher die Arzneimittelzusammensetzung zur Behandlung oder Vorbeugung von systolischem Herzversagen bestimmt ist.

6. Verwendung nach Anspruch 5, bei welcher die Arzneimittelzusammensetzung zur Vorbeugung von systolischem Herzversagen bestimmt ist.

7. Verwendung nach Anspruch 1 bis 6, bei welcher die galenische Formulierung der Arzneimittelzusammensetzung eine Tablette, eine trinkbare Lösung, eine Kapsel, ein Zäpfchen oder eine injizierbare Formulierung ist.

8. Verwendung nach einem der Ansprüche 1 bis 7, bei welcher die galenische Formulierung der Arzneimittelzusammensetzung eine Tablette ist.

9. Verwendung nach einem der Ansprüche 1 bis 7, bei welcher die galenische Formulierung der Arzneimittelzusammensetzung eine trinkbare Lösung ist.

10. Verwendung nach einem der Ansprüche 1 bis 9, bei welcher die Arzneimittelzusammensetzung nach einer ein- oder mehrstufigen täglichen Verabreichungsvorschrift zu verabreichen ist.

11. Verwendung nach Anspruch 10, bei welcher Cilobradin oder dessen pharmazeutisch annehmbares Salz mit zwischen 0,01 und 20 mg/kg Körpergewicht zu verabreichen ist.

12. Verwendung nach Anspruch 10, bei welcher Cilobradin oder dessen pharmazeutisch annehmbares Salz mit zwischen 0,05 und 5 mg/kg Körpergewicht zu verabreichen ist.

13. Verwendung nach Anspruch 10, bei welcher Cilobradin oder dessen pharmazeutisch annehmbares Salz mit zwischen 0,1 und 2,5 mg/kg Körpergewicht zu verabreichen ist.

14. Verwendung nach Anspruch 10, bei welcher Cilobradin oder dessen pharmazeutisch annehmbares Salz mit zwischen 0,1 und 1 mg/kg Körpergewicht zu verabreichen ist.

15. Verwendung nach Anspruch 10, bei welcher Cilobradin oder dessen pharmazeutisch annehmbares Salz mit zwischen 0,1 und 0,75 mg/kg Körpergewicht zu verabreichen ist.

16. Verwendung nach einem der Ansprüche 1 bis 15, bei welcher die Behandlung oder Vorbeugung von Herzversagen in Kombination mit anderen therapeutischen Wirkstoffen zur Behandlung oder Vorbeugung von Herzversagen wie Diuretika, Herzglykosiden, ACE-Hemmern, ARBs, Vasodilatatoren, Beta-Blockern oder Inotropika vorgenommen wird.

## Revendications

1. Utilisation de la cilobradine ou d'un sel pharmaceutiquement acceptable de celle-ci pour la préparation d'une composition pharmaceutique pour le traitement ou la prévention de l'insuffisance cardiaque.

2. Utilisation selon la revendication 1 où la composition pharmaceutique est destinée à la prévention de l'insuffisance cardiaque.

3. Utilisation selon la revendication 1 où la composition pharmaceutique est destinée au traitement ou à la prévention de l'insuffisance cardiaque diastolique.

4. Utilisation selon la revendication 3 où la composition pharmaceutique est destinée à la prévention de l'insuffisance cardiaque diastolique.

5. Utilisation selon la revendication 1 où la composition pharmaceutique est destinée au traitement ou à la prévention de l'insuffisance cardiaque systolique.

6. Utilisation selon la revendication 5 où la composition pharmaceutique est destinée à la prévention de l'insuffisance cardiaque systolique.

7. Utilisation selon l'une quelconque des revendications 1 à 6 où la formulation galénique de la composition pharmaceutique est un comprimé, une solution à boire, une capsule, un suppositoire ou une formulation injectable.

8. Utilisation selon l'une quelconque des revendications 1 à 7 où la formulation galénique de la composition pharmaceutique est un comprimé.

9. Utilisation selon l'une quelconque des revendications 1 à 7 où la formulation galénique de la composition pharmaceutique est une solution à boire.

10. Utilisation selon l'une quelconque des revendications 1 à 9 où la composition pharmaceutique est destinée à être administrée selon un schéma d'application quotidien à une étape ou à étapes multiples.

11. Utilisation selon la revendication 10 où la cilobradine, ou son sel pharmaceutiquement acceptable, est destinée à être administrée à raison de 0,01 à 20 mg/kg de poids corporel.

12. Utilisation selon la revendication 10 où la cilobradine, ou son sel pharmaceutiquement acceptable, est destinée à être administrée à raison de 0,05 à 5 mg/kg de poids corporel.

13. Utilisation selon la revendication 10 où la cilobradine, ou son sel pharmaceutiquement acceptable, est destinée à être administrée à raison de 0,1 à 2,5 mg/kg de poids corporel.

14. Utilisation selon la revendication 10 où la cilobradine, ou son sel pharmaceutiquement acceptable, est destinée à être administrée à raison de 0,1 à 1 mg/kg de poids corporel.

15. Utilisation selon la revendication 10 où la cilobradine, ou son sel pharmaceutiquement acceptable, est destinée à être administrée à raison de 0,1 à 0,75 mg/kg de poids corporel.

16. Utilisation selon l'une quelconque des revendications 1 à 15 où le traitement ou la prévention de l'insuffisance cardiaque est réalisé en combinaison avec d'autres agents thérapeutiques pour le traitement ou la prévention de l'insuffisance cardiaque, comme des diurétiques, des glycosides cardiaques, des inhibiteurs d'ECA, des BRA, des vasodilatateurs, des bêta-bloquants ou des inotropes.
